Europäisches Patentamt

European Patent Office

Office européen des brevets

(19)

(11) EP 0 732 926 B1

(12) EUROPÄISCHE PATENTSCHRIFT

(45) Veröffentlichungstag und Bekanntmachung des Hinweises auf die Patenterteilung:
**19.02.2003 Patentblatt 2003/08**

(21) Anmeldenummer: **95903322.6**

(22) Anmeldetag: **06.12.1994**

(51) Int Cl.⁷: **A61K 31/46**, A61K 31/407, A61P 39/02

(86) Internationale Anmeldenummer:
**PCT/EP94/04049**

(87) Internationale Veröffentlichungsnummer:
**WO 95/015756 (15.06.1995 Gazette 1995/25)**

(54) **PHARMAZEUTISCHE FORMULIERUNG ZUR PROPHYLAXE BZW. VORBEHANDLUNG EINER VERGIFTUNG DURCH PHOSPHORORGANISCHE CHOLINESTERASEHEMMER**

PHARMACEUTICAL FORMULATION FOR PREVENTING OR PRE-TREATING POISONING BY ORGANOPHOSPHORIC CHOLINESTERASE INHIBITORS

FORMULATION PHARMACEUTIQUE POUR LA PROPHYLAXIE OU LE PRETRAITEMENT D'UN EMPOISONNEMENT PAR DES INHIBITEURS ORGANOPHOSPHORES DE LA CHOLINESTERASE

(84) Benannte Vertragsstaaten:
**AT BE CH DE DK ES FR GB GR IE IT LI LU NL PT SE**
Benannte Erstreckungsstaaten:
**SI**

(30) Priorität: **10.12.1993 DE 4342173**

(43) Veröffentlichungstag der Anmeldung:
**25.09.1996 Patentblatt 1996/39**

(73) Patentinhaber:
• **LTS Lohmann Therapie-Systeme AG**
  **56626 Andernach (DE)**
• **ISRAEL INSTITUTE FOR BIOLOGICAL RESEARCH**
  **70450 Ness-Ziona (IL)**

(72) Erfinder:
• **HILLE, Thomas**
  **D-56564 Neuwied (DE)**
• **MÜLLER, Walter**
  **D-56564 Neuwied (DE)**
• **ASMUSSEN, Bodo**
  **D-22949 Ammersbek (DE)**
• **LEVY, Aharon**
  **Israel (IL)**
• **MESHULAM, YACOV**
  **Ramat-Gan (IL)**

(74) Vertreter: **Flaccus, Rolf-Dieter, Dr.**
**Patentanwalt**
**Bussardweg 10**
**50389 Wesseling (DE)**

(56) Entgegenhaltungen:
• **NEUROCHEM. INT. (UNITED KINGDOM), 1991, VOL. 18, NO. 2, PAGE(S) 265-273, Fosbraey P. et al 'Effect of acute physostigmine -hyoscine pretreatment on the neurochemical changes produced by soman in the guinea-pig'**
• **DRUG CHEM TOXICOL,, VOL. 14, NO. 1-2, PAGE(S) 1-20, 1991 ANDERSON D R et al 'EFFECTS OF SUBACUTE PRETREATMENT WITH CARBAMATE TOGETHER WITH ACUTE ADJUNCT PRETREATMENT AGAINST NERVE AGENT EXPOSURE'**
• **FUNDAM APPL TOXICOL,, VOL. 17, NO. 4, PAGE(S) 782-789, 1991 BREDOW J et al 'EFFICACY EVALUATION OF PHYSOSTIGMINE AND ANTICHOLINERGIC ADJUNCTS AS A PRETREATMENT FOR NERVE AGENT INTOXICATION'**
• **J AM COLL TOXICOL,, VOL. 10, NO. 2, PAGE(S) 215-222, 1991 SOLANA R P et al 'EFFICACY COMPARISON OF TWO CHOLINOLYTICS SCOPOLAMINE AND AZAPROPHEN WHEN USED IN CONJUNCTION WITH PHYSOSTIGMINE AND PYRIDOSTIGMINE FOR PROTECTION AGAINST ORGANOPHOSPHATE EXPOSURE'**

- DRUG CHEM TOXICOL,, VOL. 14, NO. 3, PAGE(S) 265-282, 1991 HARRIS L W et al 'PHYSOSTIGMINE ALONE AND TOGETHER WITH ADJUNCT PRETREATMENT AGAINST SOMAN SARIN TABUN AND VX INTOXICATION'
- DRUG CHEM TOXICOL,, VOL. 12, NO. 3-4, PAGE(S) 197-220, 1989 SOLANA R P et al 'COMPARING THE RESPONSE SURFACES OF TWO CHOLINOLYTICS WHEN USED IN COMBINATION WITH PHYSOSTIGMINE AS A PRETREATMENT AGAINST ORGANOPHOSPHATE CHALLENGE'
- PHARMACOL BIOCHEM BEHAV,, VOL. 31, NO. 3, PAGE(S) 633-640, 1988 LIM D K et al 'PREVENTION OF SOMAN TOXICITY AFTER THE CONTINUOUS ADMINISTRATION OF PHYSOSTIGMINE'

**Beschreibung**

Die Erfindung betrifft eine pharmazeutische Formulierung zur Prophylaxe bei Vergiftungsgefahr durch phosphororganische Cholinesterasehemmer in Gefährdungsbereichen mit Gifteinsatz insb. im landwirtschaftlichen oder militärischen Bereich.

Mit der vorliegenden Erfindung sollen pharmazeutische Formulierungen zur Verfügung gestellt werden, die zur prophylaktischen Behandlung von Vergiftungen durch phosphororganische Cholinesterasehemmer geeignete Wirkstoffe in kontrollierter Weise freisetzen. Zu den phosphororganischen Cholinesterasehemmern gehören Ester von Phosphorsäurederivaten wie z.B. Nitrostigmin (= Diethyl-(4-nitrophenyl)-thiophosphat, besser unter den Bezeichnungen Parathion oder E 605) bekannt, aber auch Tabun sowie die Phosphonsäurederivate Sarin, Soman und VX.

Cholinesterasehemmende Phosphorsäureester werden unter anderem in der Landwirtschaft als Insektizide eingesetzt. Da sie auch für Menschen toxisch sind, besteht eine grundsätzliche Gefahr für Leib und Leben von Mitarbeitern im agrikulturellen Bereich; dies umso mehr, als diese organischen Phosphorsäureester auch über die Haut aufgenommen werden können. Gegenüber den Insektiziden zeichen sich die zu den sogenannten Nervenkampfstoffen zählenden Verbindungen Tabun, Sarin, Soman und VX durch eine besonders hohe Toxizität aus. Alle dieser Verbindungen sind mehr oder weniger starke Hemmstoffe der Acetylcholinesterase, eines Enzyms, das physiologischerweise die Wirkung der an bestimmten Nervenendigungen freigesetzten Überträgersubstanz Acetylcholin beendet. Die Mehrzahl der durch Cholinesterasehemmer verursachten Vergiftungssymptome ist durch eine Überschwemmung mit körpereigenem Acetylcholin bedingt.

Die medikamentöse Grundbehandlung einer solchen Vergiftung besteht in der Verabreichung des Parasympathikolytikums Atropin, wodurch die überschießenden muskarinischen Acetylcholin-Wirkungen (z.B. Sekretionssteigerung in den Atemwegen, Bronchospasmus, Hemmung des zentralnervösen Atemantriebs) blockiert werden. Zur Normalisierung der überschießenden nikotinischen Acetylcholin-Wirkungen (z.B. Hemmung der Erregungsübertragung an den Synapsen motorischer Nerven zur Atem- und übrigen Skelettmuskulatur bis zur vollständigen peripheren Muskellähmung) steht kein geeigneter Antagonist zur Verfügung. Die peripher ausgelöste Muskellähmung läßt sich nur mit Oximen wie z.B. Pralidoxim (PAM) oder Obidoxim (Toxogonin®) aufheben, deren Wirkungsmechanismus in einer Reaktivierung der gehemmten Acetylcholinesterase besteht.

Einige phosphorische Cholinesterasehemmer zeichnen sich dadurch aus, daß sie nach Anlagerung an die Acetylcholinesterase Alkylreste abspalten, wodurch sich die Bindung stabilisiert. ("Alterung"). Der gealterter Esterase-Hemmstoff-Komplex läßt sich durch Oxime nicht reaktivieren. Bei Vergiftungen mit dem Nervenkampfstoff Soman tritt die Alterung bereits nach 2 bis 5 Minuten ein. Die Therapie mit Atropin und Oximen ist bei der Soman-Vergiftung völlig unzureichend. Die Wirksamkeit von Atropin und Oximen kann jedoch durch Vorbehandlung mit indirekten Parasympathikomimetika, z.B. Carbaminsäureester wie Pyridostigmin und Physostigmin wesentlich verbessert werden. Carbaminsäureester hemmen die Acetylcholinesterase in ähnlicher Weise wie Phosphorsäureester. Die Bindung ist allerdings kurzfristiger und voll reversibel. Für die Schutzwirkung von Carbamaten dürfte ausschlaggebend sein, daß sie in geeigneter Dosierung einen Teil der Acetylcholinesterase hemmen und damit dem Zugriff der stärker und anhaltend hemmenden Phosphor- und Phosphonsäureester entziehen, wenn die Vorbehandlung rechtzeitig erfolgte.

Auch die Behandlung einer Vergiftung durch phosphororganische Insektizide erfordert in jedem Fall ärztliche Betreuung, die rasch eingeleitet werden muß. Weil rasche ärztliche Hilfe im Falle von Erntearbeitern nicht immer gegeben ist, besteht Bedarf an Medikamenten, die prophylaktisch einer Intoxikation entgegenwirken. Der Einsatz von Carbaminsäureestern für diesen Zweck ist beschrieben (Leadbeater, L. Chem. in Brit. 24, 683, 1988). Gleiches gilt für die Wirksamkeit von Carbaminsäurestern zur Vorbehandlung einer Soman-Vergiftung im Tierexperiment (Fleischer, J. H., Harris, L.W. Biochem. Pharmacol. 14, 641, 1965, Berry, W.K., Davies, D.R. Biochem. Pharmacol, 19, 927, 1970). Prophylaktisch anzuwendende Arzneimittel dürfen in wirksamer Dosierung Reaktionsvermögen und Leistungsfähigkeit nicht beeinträchtigen. Die therapeutische Breite der Carbaminsäureester ist allerdings gering. Mit Physostigmin läßt sich zwar eine höhere Schutzwirkung erreichen als mit Pyridostigmin, die Nebenwirkungen sind jedoch höher.

DE-OS 41 15 558 beschreibt ein prophylaktisches Antidot, bestehend aus einer Kombination aus Pyridostigmin oder Physostigmin und N-methyl-4-piperidyl-1-phenylcyclopentancarboxylat-Hydrochlorid oder Arpenal, Sycotrol, Carmiphen oder Benaktyzin und zusätzlich zwingend einem Tranquilizer, nämlich Diazepam oder Clonazepam. Die unerwünschten Wirkungen von Physostigmin oder Pyridostigmin lassen sich daher nicht allein durch die aufgezählten Parasympathikolytika unterdrücken, weshalb zusätzlich Tranquilizer gegeben werden, deren Nebenwirkungsprofit ebenfalls problematisch ist.

Der Erfindung liegt die Aufgabe zugrunde, die prophylaktische Gabe von Carbaminsäureester oder anderen indirekten Parasympathikomimetika in einer Dosierung zu ermöglichen, die einen ausreichenden Schutz gegen phosphororganische Cholinesterasehemmer ohne unerwünschte Begleiterscheinungen bewirkt. Diese Aufgabe wird erfindungsgemäß gelöst durch eine pharmazeutische Formulierung mit einer Wirkstoffkombination aus mindestens einem Parasympathikomimetikum und mindestens einem Parasympathikolytikum gemäß Kennzeichen von Anspruch 1. Diese Lösung ist besonders bemerkenswert, da das Parasympathikolytikum - wie gezeigt werden kann - nicht nur zum Vergiftungsschutz beiträgt, sondern die unerwünschten Wirkungen des Parasympathikomimitikums sicher unterdrückt.

Zwar sind Tierexperimente zur Bekämpfung bzw. Behandlung von Somanvergiftungen aus 1988 bzw. 1991 bekannt (Pharmacology Biochemistry & Behavior, Vol. 31, No. 3, 1988, pp. 633-640, Lim, D.K. et al.; Drug and Chemical Toxicology, Vol. 14, No. 1-2, 1991, pp. 1-20, Anderson, D.R. et al.), bei denen Physostigmin über längere Zeit (4d bzw. 7d und auch 14d) mittels einer implantierten Minipumpe vor der Einwirkung von Soman durch eine intramuskuläre Verabreichung von Scopolamin kurzzeitig (10 bzw. 30 min.) vor dem Somanangriff oder auch danach verabreicht wurden. Dabei wurde festgellt, daß die Überlebenschance der Tiere mit einer solchen Behandlung erhöht ist. Auch wird bereits von Anderson angegeben, daß eine Verabreichung von sowohl Physostigmin als auch Scopolamin 10 min. vor der Somanexposition für besonders wirksam gehalten wird.

Eine im Humanbereich brauchbare Applikationsform, bei der eine anhaltende Schutzwirkung bei Gefährdung durch phosphororganische Cholinesterasehemmer mittels einer brauchbaren Applikationsform, wie in den Patentsprüchen definiert, erreicht werden kann, wurde jedoch bislang nicht in Erwägung gezogen.

Im Gegensatz zu direkten Parasympathikomimetika wirken indirekte Parasympathikomimetika nicht als Agonisten am Acetylcholin-Rezeptor. Sie verhindern vielmehr den Abbau von Acetylcholin dadurch, daß sie das Enzym Acetylcholinesterase hemmen, z.B. Carbaminsäurederivate wie Physostigmin, Heptylphysostigmin, Neostigmin und Pyridostigmin. Da die Wirkung dieser Substanzen durch die Übertragung der Carbaminsäure zustandekommt, sind Racemate ebenso wirksam wie die genuinen Enantiomere. Daher schließt die Erfindung auch Racemate ein. Ferner kommen auch andere Acetylcholinesterasehemmer, wie z.B. Galanthamin oder Tetrahydroacridin bzw. Velnacridin in Frage, deren Wirkungsmechanismus nicht auf der Übertragung von Carbaminsäure beruht. Unter dem Begriff Parasympathikolytikum werden Stoffe verstanden, die eine Affinität zu muskarinischen Acetylcholin-Rezeptoren besitzen, ohne aber eine Wirkung auszulösen. Ohne die Erfindung einzuschränken, seien genuine Alkaloide wie Scopolamin (L-Hyoscin) und L-Hyoscyamin, ihre Racemate, z.B. Atropin, oder ihre halbsynthetischen Derivate, z.B. Homatropin oder N-Butylscopolamin, erwähnt. Ferner kommen vollsynthetische Parasympathikolytika wie Benzatropin oder Benzetinmid in Frage. Andere Parasympathikomimetika bzw. - lytika sind dem Fachmann bekannt. Entscheidend ist nicht nur die Auswahl der Arzneistoffe, sondern auch, daß diese kontrolliert und aufeinander abgestimmt aus der Arzneiform freigesetzt werden.

Arzneiformen, die Wirkstoffe kontrolliert freisetzen, sind im Stand der Technik bereits bekannt. Die Verabreichung pharmazeutisch wirksamer Verbindungen mittels solcher Formulierungen kann oral oder anderweitig, z.B. parenteral erfolgen.

Die im Rahmen der vorliegenden Erfindung geeigneten Formulierungen zur oralen Verabreichung sollen kurz beschrieben werden. In einer solchen Formulierung ist der pharmazeutische Wirkstoff in einer semiperablen Membran eingekapselt, wie z.B. in Zelluloseacetat. Mit einem Bohrer oder einem Laserstrahl wird in das Kapselmaterial ein winziges Loch gebohrt. Im Magen-Darm-Trakt des Patienten wird durch das Kapselmaterial Wasser absorbiert. Der pharmazeutische Wirkstoff wird durch osmotischen Druck in der gewünschten, allmählichen, konstanten und kontrollierten Weise durch die kleine Öffnung getrieben. Solche Systeme sind in den US-Patenten 3 760 805, 3 760 806, 3 764 984, 3 845 770, 3 916 899 und 3 987 790 beschrieben. In diesen Systemen können die pharmazeutischen Wirkstoffe in fester Form oder absorbiert an Ionenaustauscher-Harzen vorliegen.

Ein anderes System zur oralen Verabreichung gemäß der vorliegenden Erfindung wird von Sheth und Leeson im US-Patent 4 137 300 beschrieben. Dieses Patent beschreibt eine Formulierung, die eine Wachsmatrix enthält.

Die Wirkstoffe der vorliegenden Erfindung werden mittels entsprechender Formulierung auf passende und geeignete Weise verabreicht. Die festen Wirkstoffe können in Lösung oder als Suspension verabreicht werden. Das Lösungs- oder Suspensionsmedium kann wässrig oder organisch sein.

Geeignete Lösungs- oder Suspensionsmedien für Arzneistoffe sind z.B. Wasser, Silikonfluid oder Mineralöl.

Um die Verabreichung einer Verbindung mittels einer Formulierung wie vorstehend beschrieben zu vereinfachen, kann dem System ein Fließverbesserer zugesetzt werden. Einige geeignete Fließverbesserer für orale Formulierungen umfassen beispielsweise Polyethylenglykol, Hydroxypropylmethylcellulose und Zucker.

Formulierungen, die zur Applikation von Wirkstoffen in Frage kommen, sind solche, die eine Depotwirkung des Wirkstoffes gestatten. Hierbei wird die Formulierung als Injektionslösung auf nicht wässriger Grundlage appliziert. Die möglichen Lösungsmittel sind dem Fachmann bekannt. Ohne die Erfindung einzuschränken, seien als Beispiele die vegetabilischen Öle erwähnt, die einzelne Pharmakopöen vorschreiben. Erdnußöl, Olivenöl, Mandelöl, Sonnenblumenöl, Sojabohnenöl und Sesamöl stehen im Vordergrund. Rizinusöl zeigt oftmals eine besonders günstige Löslichkeit für Arzneimittel, daneben sind auch Öle tierischen Ursprungs geeignet.

Die Öle sind physiologisch indifferent und gut verträglich. Voraussetzung hierfür ist, daß sie besonders gereinigt sind und niedrige Säure- und Peroxidzahlen aufweisen. Da eine intravenöse Applikation wegen der fehlenden Mischbarkeit mit dem Blutserum nicht möglich ist und zur Lungenembolie führen kann, ist lediglich ihre Anwendung für intramuskuläre und subkutane Injektionspräparate möglich. Ölige Lösungen und Suspensionen verbleiben recht lange am Ort der Applikation (oft bis zu 1 Monat) und geben die Wirkstoffe protahiert frei.

Der Nutzen einer Wirkstoffkombination von Parasympathikomimetikum (a) und Parasympathikolytikum (b) mit kontrollierter Wirkstofffreisetzung, die zu einer länger anhaltenden Verfügbarkeit von (a) und (b) im Plasma führt, wird durch die folgenden Beispiele verdeutlicht:

Tierexperimentelle Wirksamkeitsprüfung:

Gegenüber einer Somanvergiftung an Meerschweinchen wurde die Schutzwirkung von Pyridostigmin und Physostigmin allein sowie in Kombination mit Scopolamin untersucht. Jeweils 6 bis 10 Tiere erhielten 24 h vor der Soman-Belastung ein Pyridostigmin ($3 \, cm^2$/kg)- oder Physostigmin-Hautpflaster ($1,5 \, cm^2$/kg). Nach 24stündiger Anwendung des Physostigmin-Hautpflasters wurden Plasmakonzentrationen von $0,9 \pm 0,3$ ng/ml (Mittelwert $\pm$ SEM; n = 4) gemessen. Die Cholinesteraseaktivität im Gesamtblut war bei Anwendung des größeren Pyridostigmin-Hautpflasters um $38 \pm 4\%$ gehemmt, bei Anwendung des kleineren Physostigmin-Hautpflasters um $48 \pm 10\%$. Zur Prüfung der zusätzlichen Schutzwirkung von Scopolamin wurde entweder ein handelsübliches transdermales therapeutisches System (Scopoderm® TTS) angewendet oder den Tieren wurden osmotische Minipumpen (Alzet®) mit einer Freisetzungsrate von 9 bis 10 ng Scopolaminhydrobromid pro kg Körpergewicht und Stunde subkutan implantiert. Die nach Anwendung des Pyridostigmin- bzw. Physostigminhautpflasters und Soman-Belastung mit $1,5 \, LD_{50}$ intramuskulär erzielten Ergebnisse zeigt Tabelle 1.

Die Physostigmin-Vorbehandlung ist nicht nur bei einer Soman- sondern auch bei einer Sarin-Vergiftung wirksam: Nach transdermaler Physostigmin-Scopoderm®-TTS-Vorbehandlung und Belastung mit $1,5 \, LD_{50}$ Sarin überlebten 9 von 10 Meerschweinchen ohne zusätzliche Postexpositionstherapie.

In einer zusätzlichen Versuchsreihe an Meerschweinchen wurde die Wirksamkeit der Physostigmin-Vorbehandlung mit und ohne Scopolamin gegenüber Soman bei zusätzlicher Postexpositionstherapie mit Atropinsulfat sowie Obidoximchlorid anhand des Wirksamkeitsindex (protective ratio = Quotient aus $LD_{50}$ mit Behandlung und $LD_{50}$ ohne Behandlung) ermittelt (Tabelle 2).

Tabelle 1:

| Schutzwirkung verschiedener Vorbehandlungen bei Meerschweinchen gegenüber einer Belastung mit $1,5 \, LD_{50}$ Soman i.m. ohne zusätzliche Postexpositionstherapie. | |
|---|---|
| Vorbehandlung | Letalität (24h) |
| keine | 10/10 |
| Pyridostigmin transdermal ($3 cm^2$/kg) | 6/6 |
| Pyridostigmin transdermal ($1,5 \, cm^2$/kg) + Alzet® -Scopolamin 10 $ng/kg^{-1}h^{-1}$ | 5/6 |
| Pyridostigmin transdermal ($1,5 \, cm^2$/kg) | 6/20 |
| Pyridostigmin transdermal ($1,5 \, cm^2$/kg) + Alzet® -Scopolamin 9 $ng \, kg^{-1}h^{-1}$ | 0/10 |
| Physostigmin transdermal ($1,5 \, cm^2$/kg) + Scopoderm® | 1/10 |

Tabelle 2:

| Wirksamkeit der Physostigmin- bzw. kombinierten Physostigmin-Scopolamin-Vorbehandlung bei Meerschweinchen gegenüber Soman-Belastung und zusätzlicher Postexpositionstherapie mit Atropinsulfat und Obidoximchlorid (jeweils 10 mg/kg Körpergewicht i.m. 1 m in nach Soman). | |
|---|---|
| Vorbehandlung | Wirksamkeitsindex *) (Vertrauensgrenzen) |
| Pyridostigmin transdermal ($1,5 \, cm^2$/kg) | 3,45 <br> (3,00; 3,95) |
| Pyridostigmin transdermal ($1,5 \, cm^2$/kg) + Alzet® -Scopolamin 4,5 $ng \, kg^{-1}h^{-1}$ | 3,70 <br> (3,65;4,50) |

*) Wirksamkeitsindex $= \dfrac{LD_{50} \text{ mit Behandlung}}{LD_{50} \text{ ohne Behandlung}}$

Für die kombinierte Vorbehandlung mit transdermalem Physostigmin und Scopoderm® TTS ohne Postexpositionstherapie ergaben sich in Versuchsreihen mit zwei unterschiedlichen Physostigmin-Formulierungen Wirksamkeitindices von 2,11 (1,71; 2,60) bzw. 2,27 (1,86; 2,79).

Die Pharmakokinetik von transdermal verabreichtem Physostigmin und Scopolamin wurde an Schweinen untersucht. Innerhalb von 5 bis 6 h stieg die Plasmakonzentration auf ein Plateau an, das über 72 h aufrecht erhalten blieb. Für Wirksamkeitsprüfungen an Schweinen gegenüber intravenöser Soman-Belastung wurden Physostigmin-

Hautpflaster (0,5 cm$^2$/kg) verwendet, die nach 48 h zu Plasmakonzentrationen von 1,1 ± 0,1 ng/ml (16 ± 3% Hemmung der Cholinesteraseaktivität im Gesamtblut) führten. Mit Scopoderm®-TTS wurden Scopolamin-Konzentrationen im Plasma von 0,18 ± 0,06 ng/ml (n = 9) nach 24 h erreicht. Gegenüber einer Belastung mit 2,5 LD$_{50}$ Soman ergaben sich ohne zusätzliche Postexpositionstherapie folgende Befunde (Tabelle 3):

Tabelle 3:

| Schutzwirkung der Physostigmin- bzw. Physostigmin-Scopolamin-Vorbehandlung bei Schweinen gegenüber einer Belastung mit 2,5 LD$_{50}$ Soman i.v. ohne zusätzliche Postexpositionstherapie. | | |
|---|---|---|
| Vorbehandlung | Letalität | mittlere Erholungszeit *) (min) |
| Scopoderm® TTS | 4/4 | --- |
| Physostigmin transdermal (0,5 cm$^2$/kg) | 1/4 | 146 |
| Physostigmin transdermal (0,5 cm$^2$/kg) + Scopoderm®TTS | 2/5 | 29 |

*) Erholungszeit = Zeit, bis die überlebenden Tiere stehen und laufen können.

Wurden Schweine nach transdermaler Physostigmin-Scopolamin-Vorbehandlung nicht mit 2,5 LD$_{50}$, sondern mit 4 LD$_{50}$ Soman i.v. belastet und wurde 20 s später eine Postexpositionstherapie durchgeführt (0,5 mg Atropinsulfat sowie 3 mg Obidoximchlorid/kg Körpergewicht i.m.), überlebten 3 von 5 Tieren, wobei die überlebenden höhere Physostigmin- und Scopolamin-Konzentrationen aufwiesen als die Nicht-Überlebenden. Enthielt die Postexpositionstherapie zusätzlich Loprazolam (0,2 mg/kg i.m.) überlebten alle 5 Tiere, die Erholung war allerdings bei 2 Tieren unzureichend, was die Nachteile der Benzodiazepingabe exemplarisch darstellt.

Klinische Verträglichkeitsuntersuchungen

Die Verträglichkeit von Physostigmin-Hautpflastern wurde an 11 freiwilligen Probanden (Alter 29 ± 2 Jahre) unter Doppelblindbedingungen gegenüber Placebo und zusätzlicher Anwendung von Scopoderm® TTS geprüft. Bei Physostigmin-Konzentrationen im Plasma von 0,3 ± 0,1 ng/ml nach 48 h und Scopolamin-Konzentrationen von 0,07 ± 0,01 ng/kg erwies sich Scopolamin als wirksam, die durch Physostigmin verursachten unerwünschten Wirkungen, insbesondere Übelkeit und Erbrechen, zu unterdrücken. Statistisch signifikante Verhaltens- und Leistungsfähigkeitsveränderungen ließen sich bei kombinierter Physostigmin-Scopolamin-Behandlung nicht nachweisen. Damit ist die erfindungsgemäß gestellte Aufgabe gelöst, die darin bestand, eine Arzneiform zu entwickeln, die mindestens ein Parasympathikomimetikum und mindestens eine Parasympathikolytikum enthält, ohne daß die für diese Arzneistoffe typischen Nebenwirkungen auftreten.

**Patentansprüche**

1.   Pharmazeutische Formulierung zur Prophylaxe bei Vergiftungsgefahr durch phosphororganische Choliriesterasehemmer in Gefährdungsbereichen mit Gifteinsatz insb. im landwirtschaftlichen oder militärischen Bereich, enthaltend eine Wirkstoffkombination bestehend aus zumindest einem Parasympathikomimetikum und mindestens einem Parasympathikolytikum in einer Darreichungsform für orale oder parenterale Applikation der Wirkstoffkombination mit kontrollierter und aufeinander abgestimmter Freisetzung der Wirkstoffe.

2.   Pharmazeutische Formulierung nach Anspruch 1, **dadurch gekennzeichnet, daß** sie als intramuskuläres oder subcutanes Injektionspräparat in Form einer Injektionslösung auf nicht-wässriger Grundlage vorliegt.

3.   Pharmazeutische Formulierung nach Anspruch 1, **dadurch gekennzeichnet, daß** sie in einer oral applizierbaren Form als Kapsel mit einer semipermeablen Membran-Wand mit kleiner Öffnung vorliegt.

4.   Pharmazeutische Formulierung nach Anspruch 1, **dadurch gekennzeichnet, daß** sie die Wirkstoffe bzw. Wirkstoffkombination in fester Form oder an Ionenaustauscher-Harze adsorbiert enthält.

**5.** Pharmazeutische Formulierung nach Anspruch 1, **dadurch gekennzeichnet, daß** das Parasympathikolytikum aus der Gruppe der Tropaalkaloide, deren Salzen und racemischen Gemischen ausgewählt ist.

**6.** Pharmazeutische Formulierung nach Anspruch 1, **dadurch gekennzeichnet, daß** sie in einer Darreichungsform vorliegt, bei welcher das Parasympathikomimetikum indirekt wirksam ist.

**7.** Pharmazeutische Formulierung nach Anspruch 6, **dadurch gekennzeichnet, daß** das indirekt wirksame Parasympathikomimetikum Acetylcholinesterasehemmer umfaßt.

**8.** Pharmazeutische Formulierung nach Anspruch 7, **dadurch gekennzeichnet, daß** der Acetylcholinesterasehemmer Physostigmin, Heptylphysostigmin, Neostigmin, Pyridostigmin, Tetrahydroacridin und Velnacridin sowie deren Salze und deren racemische Gemische umfaßt.

**9.** Pharmazeutische Formulierung nach Anspruch 1, **dadurch gekennzeichnet, daß** das Parasympathikolytikum Scopolamin und/oder dessen pharmazeutisch akzeptablen Salze und das Parasympathikomimetikum Physostigmin und/oder dessen pharmazeutisch akzeptablen Salze ist.

**Claims**

**1.** A pharmaceutical formulation for the prophylaxis in cases where there exists a danger of poisoning by organophosphorus cholinesterase inhibitors in danger areas involving the use of poison, especially in the agricultural or military sector, containing an active substance combination consisting of at least one parasympathomimetic and at least one parasympatholytic in an administration form for oral or parenteral application of the active substance combination with a controlled active substance release and with the release of each active substance adjusted to that of the other active substances.

**2.** Pharmaceutical formulation according to Claim 1, **characterized in that** it is present in an intramuscular or subcutaneous injection preparation in the form of an injection solution on a non-aqueous basis.

**3.** Pharmaceutical formulation according to Claim 1, **characterized in that** it is present in an orally applicable form as a capsule comprising a semipermeable membrane wall having a small opening.

**4.** Pharmaceutical formulation according to Claim 1, **characterized in that** it contains the active substances or active substance combination in solid form or adsorbed to ion exchange resins.

**5.** Pharmaceutical formulation according to Claim 1, **characterized in that** the parasympatholytic is selected from the group of tropane alkaloids, their salts and racemic mixtures.

**6.** Pharmaceutical formulation according to Claim 1, **characterized in that** it is present in an administration form wherein the parasympathomimetic is indirectly effective.

**7.** Pharmaceutical formulation according to Claim 6, **characterized in that** the indirectly effective parasympathomimetic comprises acetylcholinesterase inhibitors.

**8.** Pharmaceutical formulation according to Claim 7, **characterized in that** the acetylcholinesterase inhibitor comprises phystostigmine, heptylphysostigmine, neostigmine, pyridostigmine, tetrahydroacridine and velnacridine, as well as their salts and their racemic mixtures.

**9.** Pharmaceutical formulation according to Claim 1, **characterized in that** the parasympatholytic is scopolamine and/or its pharmaceutically acceptable salts and that the parasympathomimetic is physostigmine and/or its pharmaceutically acceptable salts.

**Revendications**

**1.** Formulation pharmaceutique prophylactique en cas de risque d'empoisonnement par des inhibiteurs organophosphores de la cholinestérase dans des zones à risque utilisant des poisons notamment dans les secteurs agricoles ou militaires, contenant une combinaison de substances actives constituées d'au moins un parasympathicomimétique et d'au moins un parasympathicolytique dans une forme galénique pour application orale ou parentérale de la combinaison de substances actives avec libération contrôlée et assortie des molécules.

2. Formulation pharmaceutique selon la revendication 1, **caractérisée en ce qu'**elle se présente comme préparation injectable intramusculaire ou sous-cutanée sous forme d'un soluté injectable sur une base non acqueuse.

3. Formulation pharmaceutique selon la revendication 1, **caractérisée en ce qu'**elle se présente sous une forme applicable par voie orale comme gélules avec une paroi de membrane semi-perméable munie d'un petit orifice.

4. Formulation pharmaceutique selon la revendication 1, **caractérisée en ce qu'**elle contient les substances actives ou la combinaison de substances actives sous forme solide ou adsorbée sur des résines échangeuses d'ions.

5. Formulation pharmaceutique selon la revendication 1, **caractérisée en ce que** le parasympathicolytique est sélectionné parmi le groupe des alcaloïdes tropaniques, leurs sels ou leurs mélanges racémiques.

6. Formulation phàrmaceutique selon la revendication 1, **caractérisée en ce qu'**elle se présente sous une forme galénique avec laquelle le parasympathicomimétique a une action indirecte.

7. Formulation pharmaceutique selon la revendication 6, **caractérisée en ce que** le parasympaticomimétique à action indirecte comprend des inhibiteurs de l'acétylcholinestérase.

8. Formulation pharmaceutique selon la revendication 7, **caractérisée en ce que** l'inhibiteur de l'acétylcholinestérase comprend la physostigmine, l'heptylphysostigmine, la néostigmine, la pyridostigmine, la tétrahydroacridine et la velnacridine ainsi que leurs sels et leurs mélanges racémiques.

9. Formulation pharmaceutique selon la revendication 1, **caractérisée en ce que** le parasympathycolytique est la scopolamine et/ou ses sels pharmaceutiquement acceptables et le parasympathicomimétique, la physostigmine et/ou ses sels pharmaceutiquement acceptables.